# EUROPEAN PATENT SPECIFICATION

(11) **EP 2 350 300 B1**
(45) Date of publication and mention of the grant of the patent: **14.12.2016**
(21) Application number: 09743854.3
(22) Date of filing: 14.10.2009
(51) Int. Cl.: C12P 7/64, C11B 1/02, C11B 1/06, D21C 3/04, C12P 19/02, C12P 19/14, C13K 1/02

(54) **PROCESS FOR THE PRODUCTION OF LIPIDS FROM BIOMASS**
VERFAHREN ZUR HERSTELLUNG VON LIPIDEN AUS BIOMASSE
PROCÉDÉ POUR LA PRODUCTION DE LIPIDES À PARTIR DE BIOMASSE

(30) Priority: 21.10.2008 IT MI20081863
(43) Date of publication of application: 03.08.2011
(73) Proprietor: ENI S.p.A., 00144 Rome (IT)
(72) Inventor: BIANCHI, Daniele, I-20020 Arese-Milano (IT); FRANZOSI, Giuliana, I-28100 Novara (IT); ROMANO, Anna Maria, I-28100 Novara (IT)
(74) Representative: Bottero, Carlo
(86) International application number: PCT/EP2009/007388
(87) International publication number: WO 2010/046051

(56) References cited:
- US-A- 2 783 146
- DAI CHUAN-CHAO ET AL: "Biodiesel generation from oleaginous yeast Rhodotorula glutinis with xylose assimilating capacity" AFRICAN JOURNAL OF BIOTECHNOLOGY, ACADEMIC PRESS, US, vol. 6, no. 18, 1 September 2007 (2007-09-01), pages 2130-2134, XP002532682 ISSN: 1684-5315 cited in the application
- DATABASE MEDLINE [Online] US NATIONAL LIBRARY OF MEDICINE (NLM), BETHESDA, MD, US; 2000, GREENWALT C J ET AL: "Ozonation and alkaline-peroxide pretreatment of wheat straw for Cryptococcus curvatus fermentation." XP002545876 Database accession no. NLM11676439 & LIFE SUPPORT & BIOSPHERE SCIENCE : INTERNATIONAL JOURNAL OF EARTH SPACE 2000, vol. 7, no. 3, 2000, pages 243-249, ISSN: 1069-9422
- DATABASE BIOSIS [Online] BIOSCIENCES INFORMATION SERVICE, PHILADELPHIA, PA, US; 20 September 2002 (2002-09-20), MOSIER NATHAN S ET AL: "Characterization of acid catalytic domains for cellulose hydrolysis and glucose degradation" XP002580543 Database accession no. PREV200200504160 & BIOTECHNOLOGY AND BIOENGINEERING, vol. 79, no. 6, 20 September 2002 (2002-09-20), pages 610-618, ISSN: 0006-3592
- KOOTSTRA A M J ET AL: "Comparison of dilute mineral and organic acid pretreatment for enzymatic hydrolysis of wheat straw" BIOCHEMICAL ENGINEERING JOURNAL, ELSEVIER, AMSTERDAM, NL LNKD- DOI:10.1016/J.BEJ.2009.04.020, vol. 46, no. 2, 1 October 2009 (2009-10-01), pages 126-131, XP026337277 ISSN: 1369-703X [retrieved on 2009-05-03]
- GREENWALT C J ET AL: "Ozonation and alkaline-peroxide pretreatment of wheat straw for Cryptococcus curvatus fermentation", LIFE SUPPORT & BIOSPHERE SCIENCE : INTERNATIONAL JOURNAL OF EARTH SPACE, COGNIZANT COMMUNICATION CORP. ELMSFORD, NY, US, vol. 7, no. 3, 1 January 2000 (2000-01-01) , pages 243-249, XP008111933, ISSN: 1069-9422
- FANTA G F ET AL: "Hydrolysis of Wheat Straw Hemicellulose with Trifluoroacetic Acid. Fermentation of Xylose with Pachysolen tannophilus", BIOTECHNOLOGY AND BIOENGINEERING, WILEY & SONS, HOBOKEN, NJ, US, vol. 26, no. 9, 1 September 1984 (1984-09-01), pages 1122-1125, XP001320278, ISSN: 0006-3592

## Description

The present invention relates to a process for the production of lipids from biomass, including at least one polysaccharide.

More specifically, the present invention relates to a process for the production of lipids from biomass including at least one polysaccharide which comprises subjecting said biomass to acid hydrolysis, subjecting the mixture obtained from said acid hydrolysis to enzymatic hydrolysis, and subjecting the sugars obtained from said hydrolysis to fermentation in the presence of at least one oleaginous yeast.

The lipids thus obtained can be advantageously used in the production of biodiesel or green diesel which can be used as such, or in a mixture with other fuels for motor vehicles.

Generally speaking, a biomass is any substance with an organic, vegetable or animal matrix, which can be destined for energy purposes, for example as raw material for the production of biofuels, or of components which can be added to fuels. Biomass can therefore form a renewable energy source as an alternative to traditional raw materials of a fossil origin normally used in the production of fuels. For this purpose, lignocellulosic biomass is particularly useful.

The production of sugars from biomass, in particular lignocellulosic biomass, is known in the art.

Lignocellulosic biomass is a complex structure comprising three main components: cellulose, hemicellulose and lignin. Their relative amounts vary according to the type of lignocellulosic biomass used. In the case of plants, for example, said amounts vary according to the species and age of the plant.

Cellulose is the greatest constituent of lignocellulosic biomass and is generally present in an amount ranging from 30% by weight to 60% by weight with respect to the total weight of the lignocellulosic biomass. Cellulose consists of glucose molecules (from about 500 to 10,000 units) bound to each other through a β-1,4 glucoside bond. The establishment of hydrogen bonds between the chains causes the formation of crystalline domains which give resistance and elasticity to vegetal fibres. In nature, it can only be found in its pure state in annual plants such as cotton and flax, whereas in ligneous plants it is always accompanied by hemicellulose and lignin.

Hemicellulose which is generally present in an amount ranging from 10% by weight to 40% by weight with respect to the total weight of the lignocellulosic biomass, appears as a mixed polymer, relatively short (from 10 to 200 molecules) and branched, made up of both sugars with six carbon atoms (glucose, mannose, galactose) and also sugars with five carbon atoms (xylose, arabinose). Some important properties of vegetal fibres are due to the presence of hemicellulose, of which the main property is that of favouring the imbibition of said vegetal fibres, when water is present, causing their swelling. Hemicellulose also has adhesive properties and therefore tends to cement or develop a horny consistency, with the consequence that said vegetal fibres become rigid and are imbibed more slowly.

Lignin is generally present in an amount ranging from 10% by weight to 30% by weight with respect to the total weight of the lignocellulosic biomass. Its main function consists in binding and cementing the various vegetal fibres with each other giving the plant compactness and resistance and also provides protection against insects, pathogen agents, lesions and ultraviolet light. It is mainly used as fuel but is also currently widely used in industry as a disperser, hardener, emulsifying agent, for plastic laminates, cartons and manufactured rubber articles. It can also be chemically treated to produce aromatic compounds, of the vanillin, syringalde-hyde, p-hydroxybenzaldehyde type, which can be used in pharmaceutical chemistry, or in the cosmetic and food industry.

In order to optimize the transformation of lignocellulosic biomass into products for energy use, subjecting said biomass to a preliminary treatment to separate the lignin and hydrolyze the cellulose and hemicellulose to simple sugars, such as, for example, glucose and xylose, is known. These sugars can be used as carbon sources in fermentation processes in the presence of microorganisms for the production of alcohols and/or lipids.

Patent application US 2008/0102176, for example, describes a method for the extraction of vegetable fats comprising: pulverizing the raw material containing cellulose in order to obtain particles with a diameter of 1-2 mm; immerging the particles in sulfuric acid at a concentration equal to 1-2% to acidify said particles in order to increase the hydrolysis of the cellulose and to adjust the pH to a value of 4.5±0.5; removing the particles acidified by the sulfuric acid and adding, in sequence, cellulase and an oleaginous yeast to the acidified particles and subjecting to fermentation for 8-9 days at a temperature of 25-30°C and a humidity of 85-90%; adding an aliphatic hydrocarbon, as solvent, to the fermentation products in order to extract the fats, obtaining an extraction blend; removing the acidified particles remaining in the extraction blend and separating the fats from the solvent by distillation, obtaining raw oil. The cellulase is preferably *Trichoderma viride* and the oleaginous yeast is *Rhodotorula glutinis.* The fats obtained can be converted to biodiesel after esterification.

Dai et al. describe the production of biodiesel from oleaginous yeasts in the article: ""Biodiesel generation from oleaginous yeast *Rhodotorula glutinis* with xylose assimilating capacity", published in "African Journal of Biotechnology" (2007), Vol. 6 (18), pages 2130-2134. In said article, the lignocellulosic biomass is ground and subjected to acid hydrolysis in the presence of sulfuric acid. The sugars thus obtained are used as carbon sources in a fermentation process in the presence of a previously selected strain of *Rhodotorula glutinis,* capable of also using pentoses, xylose in particular, with the purpose of obtaining oils which are subsequently extracted by Soxhlet extraction and subjected to transesterification in order to obtain biodiesel.

Greenwalt et al. (2000), Life Support & Biosphere Science, vol. 7, pages 243-249, discloses ozonation and alkaline-peroxide treatment as pretreatment methods for the enzymatic saccharification and subsequent fermentation of wheat straw with the oleaginous yeast Cryptococcus curvatus.

The above processes however can have various drawbacks.

In order to hydrolyze both polysaccharide components of the biomass (e.g., hemicellulose and cellulose), for example, in particular in the presence of diluted sulfuric acid (e.g., at concentrations ranging from 0-5% to 11%) it is necessary to operate at high temperatures (e.g., over 160°C). At said temperatures, there is the formation of by-products deriving from the dehydration of the sugars and from the partial depolymerization of the lignin. These by-products, in particular furfural, hydroxyl methyl furfural, phenolic compounds, can act as growth inhibitors of the microorganisms normally used in the subsequent sugar fermentation processes. Furthermore, in the presence of diluted sulfuric acid, the sugar yield, glucose in particular, is normally low (e.g., lower than 50%).

Furthermore, before subjecting the sugars to fermentation, it may be necessary to neutralize the sulfuric acid in order to adjust the pH to values acceptable for the subsequent fermentation. Neutralization is generally obtained by adding oxides and/or hydroxides such as, for example, calcium oxide, calcium hydroxide, or barium hydroxide, with the consequent formation of salts (e.g., calcium sulphate, calcium sulfate dihydrate ("gypsum"), barium sulfate) which precipitate and must therefore be separated from the sugars before the latter are subjected to fermentation. In addition, these salts must be subsequently disposed of by means of suitable treatment with a consequent increase in the production cost. Furthermore, it is impossible to recover and reuse said inorganic acid.

The Applicant has now found that the production of lipids from biomass, in particular from biomass including at least one polysaccharide, can be advantageously effected by means of a process which comprises subjecting said biomass to acid hydrolysis in the presence of an aqueous solution of at least one organic acid, at a temperature lower than or equal to 160°C, subjecting the mixture obtained from said acid hydrolysis to enzymatic hydrolysis, and subjecting the sugars obtained from these hydrolyses to fermentation in the presence of at least one oleaginous yeast.

Numerous advantages are obtained by means of this process. Said process allows, for example, a high yield of pentose and hexose sugars to be obtained, deriving from the acid hydrolysis of said biomass, which can be subsequently used as carbon sources in fermentation processes for the production of lipids. Said lipids can be advantageously used in the production of biodiesel or green diesel which can be used as such, or mixed with other fuels for motor vehicles.

Furthermore, the possibility of operating at temperature which are not high, i.e. lower than or equal to 160°C, allows the formation of by-products to be reduced, such as, for example, furfural, hydroxyl methyl furfural, phenolic compounds, which can act as growth inhibitor of the microorganisms normally used in the subsequent fermentation processes of sugars.

Another relevant advantage, lies in the fact that said process allows the organic acid to be recovered, which can then be recycled to the above process, in particular, to the acid hydrolysis of the biomass. Said recovery also allows the neutralization step to be avoided and, therefore, the production of salts and their subsequent disposal.

A further relevant advantage, is that the wet solid phase comprising cellular debris, in particular proteins and polysaccharides contained in the cell membrane of the oleaginous yeasts used, which is obtained at the end of the fermentation, can be recovered and used in the above-mentioned process, in particular it can be used in the acid hydrolysis. The acid hydrolysis of this wet solid phase allows to obtain amino acids from proteins and sugars from polysaccharides, which are subsequently sent to the fermentation step, allowing, in this way, to reduce the amount of nitrogen which must normally be added to the culture medium in which the fermentation takes place and to provide additional sugars for the fermentation.

An object of the present invention therefore relates to a process for the production of lipids from biomass including at least one polysaccharide comprising:
- subjecting said biomass to acid hydrolysis in the presence of an aqueous solution of at least one organic acid selected from alkyl- or aryl-sulfonic acids having from C₇ to C₂₀ carbon atoms, preferably from C₉ to C₁₅ carbon atoms, at a temperature ranging from 80°C to 160°C, preferably from 100°C to 150°C, obtaining a first mixture comprising a first solid phase and a first aqueous phase;
- subjecting said first mixture to enzymatic hydrolysis obtaining a second mixture comprising a second solid phase and a second aqueous phase;
- subjecting said second aqueous phase to fermentation in the presence of at least one oleaginous yeast obtaining an oleaginous cellular biomass comprising lipids.

For the purposes of the present description and of the following claims, the definition of the numerical ranges always include extremes, unless otherwise specified.

In accordance with a preferred embodiment of the present invention, said polysaccharide can be selected from cellulose, hemicellulose or mixtures thereof. Cellulose, or mixtures of hemicellulose and cellulose, are particularly preferred.

In accordance with a further preferred embodiment of the present invention, said biomass is a lignocellulosic biomass. As mentioned above, the lignocellulosic biomass includes three components: hemicellulose, cellulose and lignin.

Said lignocellulosic biomass is preferably selected from:
- products of cultures expressly cultivated for energy use (for example, miscanthus, millet, common cane), including by-products, residues and wastes of said cultures or of their processing;
- products of agricultural cultivations, forestation and silviculture, comprising wood, plants, residues and by-products of agricultural processing, forestation and silviculture;
- agri-foodstuffs by-products intended for human feeding or zootechnics;
- residues, not chemically treated, of the paper industry;
- waste products coming from the differentiated collection of solid urban waste (e.g., urban waste of a vegetable origin, paper).

In accordance with a preferred embodiment of the present invention, said biomass can be subjected to a preliminary grinding process before being subjected to acid hydrolysis. Said biomass is preferably ground until particles having a diameter ranging from 0.1 mm to 10 mm, more preferably ranging from 0.5 mm to 4 mm, are obtained. Particles having a diameter of less than 1 mm are particularly preferred.

In accordance with a preferred embodiment of the present invention, said biomass is present in the reaction mixture in an amount ranging from 5% by weight to 40% by weight, preferably from 20% by weight to 35% by weight, with respect to the total weight of the reaction mixture.

For the purposes of the present description and of the following claims, the term "reaction mixture" means the mixture comprising the biomass and the aqueous solution of said at least one organic acid.

In accordance with a preferred embodiment of the present invention, said at least one organic acid is soluble in water and extractable with an organic solvent insoluble in water.

For the purposes of the present invention and of the following claims, the term "organic acid soluble in water" means an organic acid having a solubility in distilled water, at 25°C, of at least 0.5 g/100 ml of distilled water, preferably of at least 2 g/100 ml of distilled water.

For the purposes of the present invention and of the following claims, the term "organic acid extractable with an organic solvent insoluble in water" means an organic acid which can be extracted with an organic solvent insoluble in water with a yield of at least 80%, preferably of at least 90%, said yield being calculated with respect to the total amount of organic acid present in the aqueous solution.

For the purposes of the present invention and of the following claims, the term "organic solvent insoluble in water" means an organic solvent which has a solubility in distilled water, at 25°C, lower than 4% by volume, preferably lower than 2% by volume.

In accordance with a preferred embodiment of the present invention, said alkyl- or aryl sulfonic acids can be selected from: dodecyl-sulfonic acid, para-toluene-sulfonic acid, 1-naphthalene-sulfonic acid, 2-naphthalene-sulfonic acid, 1,5-naphthalene-disulfonic acid, or mixtures thereof. Para-toluene-sulfonic acid, 2-naphthalene-sulfonic acid, 1,5-naphthalene-disulfonic acid, or mixtures thereof, are particularly preferred.

In accordance with a preferred embodiment of the present invention, said at least one organic acid is present in the aqueous solution at a concentration ranging from 0.1% by weight to 5% by weight, preferably from 0.5% by weight to 2.5% by weight, with respect to the total weight of the aqueous solution.

Said organic acid acts as catalyst for the acid hydrolysis of said biomass. In particular, when the starting biomass is a lignocellulosic biomass, said organic acid specifically acts as catalyst for the acid hydrolysis of the hemicellulose. It should be noted that the process object of the present invention, when the starting biomass is a lignocellulosic biomass, not only allows the acid hydrolysis of the hemicellulose to be obtained, but also improves the disposition of the cellulose, said cellulose remaining substantially non-hydrolyzed, for the subsequent enzymatic hydrolysis, thanks to an improved destructuring of the starting biomass.

In accordance with a preferred embodiment of the present invention, said acid hydrolysis can be carried out for a time ranging from 20 minutes to 6 hours, preferably from 30 minutes to 3 hours.

Said acid hydrolysis can be carried out in reactors known in the art, such as, for example, autoclaves, or extruders.

In accordance with a preferred embodiment of the present invention, said first solid phase comprises lignin and cellulose and said first aqueous phase comprises at least one sugar having from C₅ to C₆ carbon atoms and said at least one organic acid. Said sugar is preferably xylose. Said xylose preferably derives from the acid hydrolysis of the hemicellulose.

Said acid hydrolysis allows at least one sugar having from C₅ to C₆ carbon atoms to be obtained, in particular xylose deriving from the acid hydrolysis of hemicellulose, with a high yield. More specifically, said acid hydrolysis allows a yield of xylose higher than or equal to 80% to be obtained, said yield being calculated with respect to the total amount of xylose present in the starting biomass.

Said acid hydrolysis also allows high yields of cellulose and lignin to be obtained.

In order to recover said at least one organic acid, said first mixture can be subjected to extraction with an organic solvent soluble in water.

In accordance with a preferred embodiment of the present invention, said process also comprises subjecting said first mixture to extraction with at least one organic solvent insoluble in water. Preferably, said organic solvent insoluble in water can be selected from: halogenated hydrocarbons such as, for example, methylene chloride, monochlorobenzene, dichlorobenzene, or mixtures thereof; aromatic hydrocarbons such as, for example, toluene, xylene, or mixtures thereof; aliphatic alcohols having from C₄ to C₆ carbon atoms such as, for example, n-butanol, n-pentanol, or mixtures thereof; or mixtures thereof. Mixtures of toluene and n-butanol are particularly preferred.

Said organic solvent insoluble in water is subsequently evaporated obtaining a further solid phase comprising said at least one organic acid.

As specified above, the process object of the present invention allows said at least one organic acid to be recovered with a high yield, in particular, with a yield of at least 80%, preferably of at least 90%, said yield being calculated with respect to the total amount of organic acid present in said aqueous solution. Said at least one organic acid can therefore be subsequently reused according to the process object of the present invention. It should be noted that, when the recovery of said at least one organic acid is equal to 100%, said first aqueous phase will be free of said at least one organic acid.

In accordance with a preferred embodiment of the present invention, said process also comprises re-using said at least one organic acid in said acid hydrolysis.

For the purposes of the process object of the present invention, said enzymatic hydrolysis can be carried out according to techniques known in the art as described, for example, in United States Patents US 5,628,830, US 5,916,780 e US 6,090,595, using commercial enzymes such as, for example, Celluclast 1.5L (Novozymes), Econase CE (Rohm Enzymes), Spezyme (Genecor), Novozym 188 (Novozymes), used individually or mixed with each other.

In order to adjust the pH to values ranging from 4 to 6, preferably from 4.5 to 5.5, glacial acetic acid can be added to said first mixture, before the enzymatic hydrolysis, in an amount such as to obtain the desired pH.

In accordance with a preferred embodiment of the present invention, said second solid phase comprises lignin and said second aqueous phase comprises at least one sugar having from C₅ to C₆ carbon atoms, preferably xylose, glucose, cellobiose, or mixtures thereof. In particular, said second aqueous phase comprises xylose deriving from the acid hydrolysis of hemicellulose, glucose and cellobiose deriving from the enzymatic hydrolysis of cellulose.

In particular, this enzymatic hydrolysis allows a high glucose yield to be obtained, more specifically a glucose yield higher than or equal to 90%, said yield being calculated with respect to the total amount of glucose present in the staring biomass.

The amounts of sugars contained in the starting biomass as also the amounts of sugars obtained after hydrolysis (acid and/or enzymatic hydrolysis), can be determined by means of known techniques in the art such as, for example, High Performance Liquid Chromatography-HPLC.

Said second solid phase and said second aqueous phase can be separated by techniques known in the art such as, for example, filtration, centrifugation. Said phases are preferably separated by filtration.

Said second solid phase, comprising lignin, can be upgraded as fuel, for example as a fuel for producing the energy necessary for sustaining the treatment processes of the biomass.

In accordance with a preferred embodiment of the present invention, said fermentation can be carried out at a temperature ranging from 20°C to 40°C, preferably from 25°C to 35°C.

In accordance with a preferred embodiment of the present invention, said fermentation can be carried out for a time ranging from 3 days to 10 days, preferably from 4 days to 8 days.

In accordance with a preferred embodiment of the present invention, said fermentation can be carried out at a pH ranging from 4.5 to 6.5, preferably from 5 to 6. In order to maintain the pH within the desired ranges, an aqueous solution of at least one inorganic base such as, for example, sodium hydroxide, potassium hydroxide, calcium hydroxide, magnesium hydroxide, or mixtures thereof, can be added to the culture medium used for the fermentation, in an amount which is such as to obtain the desired pH.

In accordance with a preferred embodiment of the present invention, said oleaginous yeast can be selected from: *Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Pichia stipitis, Torulopsis sp*..

In accordance with a preferred embodiment of the present invention, said fermentation is a feed-batch fermentation.

Before being used in said fermentation, said oleaginous yeast is preferably grown in a culture medium containing xylose, cellobiose, glucose, or mixtures thereof, at a concentration preferably ranging from 1% by weight to 2% by weight with respect to the total weight of said culture medium.

Said fermentation can be advantageously carried out in fermentors known in the art, in the presence of culture mediums normally used for the purpose comprising nutrients such as, for example, nitrogen, potassium phosphate, magnesium, salts, vitamins.

In accordance with a preferred embodiment of the present invention, said process also comprises subjecting said oleaginous cellular biomass to mechanical treatment.

Said mechanical treatment can be effected using equipment and techniques which are known in the art.

Said mechanical treatment can be advantageously effected, for example, using high-pressure homogenizers, preferably at a pressure ranging from 300 bar to 500 bar (e.g., Polytron homogenizer), or using a French press cell.

A mechanical treatment is preferably effected using a French press cell, operating at a pressure ranging from 20 Kpsi to 50 Kpsi, preferably from 35 Kpsi to 45 Kpsi.

In order to concentrate the oleaginous cellular biomass, before being subjected to mechanical treatment, said biomass can be subjected to centrifugation. Said centrifugation can be effected for a time ranging from 5 minutes to 30 minutes, preferably from 15 minutes to 25 minutes, at a rotation rate ranging from 3,000 rpm to 9,000 rpm, preferably from 4,000 to 8,000 rpm.

In order to deactivate the lipolytic enzymes (e.g., lipase), said oleaginous cellular biomass, before being subjected to mechanical treatment, can be subjected to thermal treatment. Said thermal treatment can be carried out at a temperature ranging from 70°C to 90°C, preferably from 75°C to 85°C, for a time ranging from 30 minutes to 3 hours, preferably from 1 to 2 hours.

In accordance with a preferred embodiment of the present invention, said process also comprises subjecting said oleaginous cellular biomass to centrifugation, after mechanical treatment.

In accordance with a preferred embodiment of the present invention, said centrifugation can be carried out for a time ranging from 5 minutes to 30 minutes, preferably from 10 minutes to 25 minutes, at a rotation rate ranging from 3,000 rpm to 9,000 rpm, preferably from 4,000 rpm to 8,000 rpm.

At the end of the centrifugation, the following phases are obtained:
(i) an oily phase comprising lipids;
(ii) an aqueous phase comprising traces of non-separated lipids and non-fermented sugars;
(iii) a wet solid phase comprising cellular debris and traces of non-separated lipids.

The process object of the present invention allows the lipids to be recovered with a yield ranging from 50% to 90%, preferably from 55% to 80%, said yield being calculated with respect to the total amount of lipids present in the oleaginous cellular biomass obtained after fermentation.

The lipids included in said oily phase (i) are preferably triglycerides, more preferably esters of glycerol with fatty acids having from C₁₄ to C₂₀ carbon atoms such as, for example, palmitic acid, stearic acid, oleic acid, α-linoleic acid, in an amount higher than or equal to 80% by weight, preferably higher than or equal to 90% by weight, with respect to the total weight of the lipids. Other lipids which can be present in said oily phase are: phospholipids, monoglycerides, diglycerides, or mixtures thereof.

The amount of lipids contained in the oleaginous cellular biomass obtained after fermentation, as also the amount of lipids contained in said oily phase, can be determined by means of techniques known in the art such as, for example, the colorimetric method which is based on the reaction of lipids with phosphoric acid and phospho-vanillin: further details relating to this method can be found, for example, in the following article: "Chemical Basis of the Sulpho-phospho-vanillin Reaction for Estimating Total Serum Lipids", J. A. Knight et al., published in "Clinical Chemistry" (1972), Vol. 18, No. 3, pages 199-202.

As already specified above, the wet solid phase (iii) comprising cellular debris, in particular proteins and polysaccharides contained in the cell membrane of the oleaginous yeast used, can be recovered and used in the process object of the present invention, in particular it can be sent to acid hydrolysis. The proteins and polysaccharides of the membrane are then hydrolyzed to amino acids and simple sugars (for example, glucose, mannose) which can then be used as nitrogen and carbon source in the subsequent fermentation. It should be outlined the fact that the recovery and use of said wet solid phase (iii) allows nitrogen to be supplied to the culture medium used for the fermentation, consequently reducing the amount of nitrogen normally added to the culture medium in which the fermentation takes place and providing additional sugars for the fermentation.

In accordance with a preferred embodiment of the present invention, said process also comprises sending said wet solid phase (iii) to acid hydrolysis. In this case, said first aqueous phase, in addition to said at least one sugar having from C₅ to C₆ carbon atoms and said at least one organic acid, wil comprise amino acids and simple sugars (for example, glucose and mannose) deriving from the acid hydrolysis of the proteins and of the polysaccharides of the membrane; whereas said first solid phase will comprise, in addition to cellulose and lignin, non-hydrolyzed cellular debris.

In order to obtain a higher yield of lipids, i.e. a yield higher than 90%, said aqueous phase (ii) and/or said wet solid phase (iii) can be subjected to extraction with a solvent, or with an alcohol/solvent mixture.

In accordance with a preferred embodiment of the present invention, said process also comprises subjecting said aqueous phase (ii) and/or said wet solid phase (iii) to extraction with at least one solvent which can be selected from aliphatic hydrocarbons having from C₃ to C₁₀ carbon atoms such as, for example, pentane, n-hexane, octane, or mixtures thereof; or with a mixture comprising at least one aliphatic alcohol having from C₃ to C₅ carbon atoms which can be selected from, for example, iso-propanol, n-butanol, or mixtures thereof, and at least one aliphatic hydrocarbon having from C₃ to C₁₀ carbon atoms selected from those described above. Said organic solvent and/or said mixture, are subsequently evaporated obtaining a further oily phase comprising lipids, a further aqueous phase comprising non-fermented sugars and a further wet solid phase comprising cellular debris.

As described above for the wet solid phase (iii), said further wet solid phase obtained after said extraction, can also be recovered and sent to acid hydrolysis.

The lipids obtained according to the process of the present invention can be subjected to esterification in the presence of an alcohol having from 1 to 4 carbon atoms, preferably methanol, ethanol, and of an acid or base catalyst, in order to produce glycerol and alkyl esters, in particular methyl esters or ethyl esters (biodiesel).

Alternatively, said lipids can be subjected to hydrogenation/deoxygenation in the presence of hydrogen and a catalyst in order to produce green diesel. Hydrogenation/deoxygenation processes are known in the art and are described, for example, in European patent application EP 1,728,844.

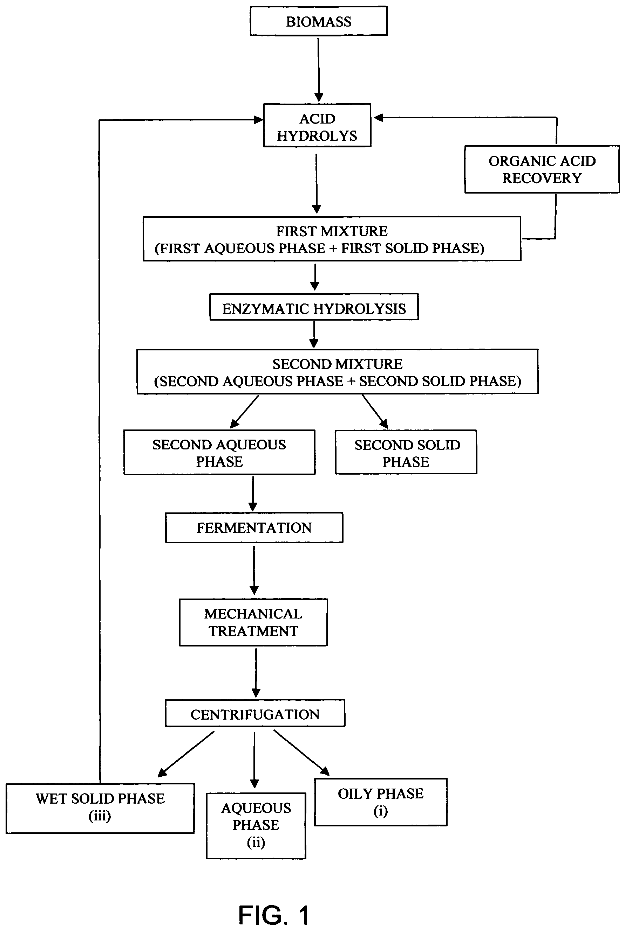

The present invention will now be described through an illustrative form with reference to Figure 1 provided hereunder.

According to a typical embodiment of the process object of the present invention, the biomass (e.g., previously ground lignocellulosic biomass) is subjected to acid hydrolysis in the presence of an organic acid (e.g., 2-naphthalene-sulfonic acid), obtaining a first mixture comprising a first aqueous phase including xylose deriving from the hydrolysis of hemicellulose and said organic acid, and a first solid phase comprising lignin and cellulose.

As represented in Figure 1, said first mixture is subjected to extraction with an organic solvent insoluble in water (e.g., a mixtures of toluene and n-butanol) in order to recover said organic acid and recycle it, after evaporation of the solvent, to said acid hydrolysis.

After extraction with the solvent, said first mixtures is subjected to enzymatic hydrolysis, in the presence of an enzyme (e.g., an aqueous solution of Celluclast 1,5L and Novozym 188) obtaining a second mixture comprising a second aqueous phase including xylose deriving from the acid hydrolysis of hemicellulose, glucose and cellobiose deriving from the enzymatic hydrolysis of cellulose, and a second solid phase including lignin.

Before being subjected to enzymatic hydrolysis, glacial acetic acid can be added to said first mixture in order to adjust the pH to values ranging from 4 to 6 (not represented in Figure 1).

Said second aqueous phase and said second solid phase are separated by filtration (not represented in Figure 1).

Said second aqueous phase is subjected to fermentation in the presence of an oleaginous yeast (e.g., *Lypomyces starkey* NRRL 1389).

At the end of the fermentation, an oleaginous cellular biomass is obtained, which is subjected to mechanical treatment (e.g., by means of a French press cell), and to subsequent centrifugation obtaining the following three phases:
(i) an oily phase comprising lipids;
(ii) an aqueous phase comprising traces of non-separated lipids and non-fermented sugars;
(iii) a wet solid phase comprising cellular debris and traces of non-separated lipids.

In order to concentrate the oleaginous cellular biomass, before being subjected to mechanical treatment, said biomass can be subjected to centrifugation (not represented in Figure 1).

In order to deactivate the lipolytic enzymes (e.g., lipase), before being subjected to mechanical treatment, said oleaginous cellular biomass can be subjected to thermal treatment (not represented in Figure 1).

As represented in Figure 1, said wet solid phase (iii) is sent to acid hydrolysis.

In order to obtain a higher yield of lipids, said aqueous phase (ii) and said wet solid phase (iii) can be subjected to extraction in the presence of a mixture comprising at least one aliphatic alcohol and at least one aliphatic hydrocarbon (e.g., a mixture of iso-propanol and n-hexane) (not represented in Figure 1).

Some illustrative and non-limiting examples are provided for a better understanding of the present invention and for its embodiment.

### EXAMPLE 1

### Acid hydrolysis of the biomass

300 g of coniferous wood previously ground (particle diameter < 1 mm) were added to a solution of 20 g of 2-naphthalenesulfonic acid in 1 1 of water.

The composition of the starting biomass was the following: 50% by weight of cellulose, 25% by weight of hemicellulose, 25% by weight of lignin, with respect to the total weight of the starting biomass.

The reaction mixture thus obtained, was maintained, under stirring, in an autoclave, at 140°C, for 1 hour, obtaining a first mixture comprising 225 g (dry weight) of a first solid phase (comprising cellulose and lignin) and 1,095 g of a first aqueous phase (comprising xylose deriving from the hydrolysis of hemicellulose and 2-naphthalene-sulfonic acid).

After cooling to 25°C, said first mixture was subjected to extraction with 1 1 of a mixture of toluene and n-butanol (3/1 by volume).

The mixture of toluene and n-butanol was subsequently separated and evaporated, at reduced pressure, obtaining a further solid phase comprising 19.6 g (dry weight) of 2-naphthalenesulfonic acid (recovery yield 98% calculated with respect to the total amount of the acid present in the aqueous solution).

After separation of the acid, glacial acetic acid was added to said first mixture until a pH of said first aqueous phase equal to 5 was obtained.

An aqueous solution of the enzyme Celluclast 1,5L (Novozymes) corresponding to 1485 FPU (Filter Paper Units) and the enzyme Novozym 188 (from Novozymes) corresponding to 18000 BGU (Beta Glucanase Units) was subsequently added. The suspension thus obtained was maintained under stirring, for 72 hours, at 45°C.

At the end of the enzymatic hydrolysis, 90 g (dry weight) of a second solid phase comprising lignin (75 g - dry weight) and non-hydrolyzed cellulose (15 g - dry weight) and 1,200 g of a second aqueous phase comprising glucose (152 g - dry weight) and xylose (72 g - dry weight), were separated by filtration.

### Fermentation

250 ml of said second aqueous phase obtained as described above, containing 200 g/l of sugars were suitably diluted with distilled water until a solution having a final concentration of sugars equal to 50 g/l was obtained.

The following nutrients were added to this solution: ammonium sulfate 0,1%, KH₂PO₄ 0,1%, MgSO₄ 7H₂O·0,005%, NaCl 0,001%, CaCl₂ 0,001%, yeast extract 0,1%. The fermentation broth (culture medium) thus obtained was inoculated in a fermentor with yeast cells (*Lypomices starkey* NRRL 1389) (inoculum equal to 2,5 g/l - dry weight) for a fermentation volume equal to 1 litre and the pH was maintained at 5.5 by the addition of an aqueous solution of NaOH 0.1 M.

The yeast cells were grown, under stirring at 500 rpm, at 30°C, aeration 0.5 l/min of sterile air.

A further 250 ml of said second aqueous phase obtained from the hydrolysis of the biomass, containing 200 g/l of sugars, suitably diluted with distilled water to obtain a solution having a final concentration of sugars equal to 50 g/l, were added to the fermentor.

After 150 hours, the yeast cells were collected and centrifuged at 7,000 rpm, for 20 minutes, obtaining 52 g (dry weight) of oleaginous cellular biomass.

### Recovery of the lipids

The oleaginous cellular biomass thus obtained was maintained at 80°C, for two hours, in order to inhibit the lipolytic enzymes and was then resuspended in 200 ml of water.

The suspension thus obtained was subjected to mechanical treatment by means of a French press cell, operating at a pressure of 39 Kpsi obtaining a cellular lysate.

Said cellular lysate was centrifuged at 7,000 rpm, for 20 minutes obtaining three phases: an oily phase insoluble in water, an aqueous phase and a wet solid phase.

The oily phase insoluble in water was separated and dried in an oven at 60°C, obtaining 19 g (dry weight) of an oily residue comprising lipids.

The aqueous phase comprising traces of non-separated lipids and non-fermented sugars and the wet solid phase comprising cellular debris and traces of non-separated lipids, were extracted with 50 ml of a mixture comprising n-hexane and isopropanol in a proportion of 3:1. The mixture of solvents was separated and evaporated, at reduced pressure, obtaining 12.2 g (dry weight) of a further oily residue comprising lipids, a further wet solid phase and a further aqueous phase.

The total amount of lipids obtained is therefore equal to 31.2 g (dry weight) (recovery yield of 60% calculated with respect to the total amount of the oleaginous cellular biomass obtained after fermentation).

The lipids were determined by means of the colorimetric method which is based on the reaction of the lipids with phosphoric acid and phospho-vanillin.

The further wet solid phase, obtained after extraction of the lipids, comprising water and cellular debris, including 42% by weight (dry weight) of polysaccharides and 45% by weight of proteins (dry weight), was separated by centrifugation from the further aqueous phase comprising non-fermented sugars.

### EXAMPLE 2

The further wet solid phase, obtained after extraction of the lipids as described in Example 1, was sent to acid hydrolysis, operating under the conditions indicated below.

42 g (dry weight) of said further wet solid phase and 260 g of coniferous wood previously ground (particle diameter < 1 mm) were added to a solution of 20 g of 2-naphthalenesulfonic acid in 1 1 of water.

The composition of the starting biomass was the following: 50% by weight of cellulose, 25% by weight of hemicellulose, 25% by weight of lignin, with respect to the total weight of the starting biomass.

The reaction mixture thus obtained, was maintained, under stirring, in an autoclave, at 140°C, for 1 hour, obtaining a first mixture comprising 198 g (dry weight) of a first solid phase (comprising cellulose, lignin and cellular debris) and 1,100 g of a first aqueous phase (comprising xylose deriving from the hydrolysis of hemicellulose, glucose, mannose and amino acids deriving from the hydrolysis of the polysaccharides and of the membrane proteins included in the cellular debris and 2-naphthalenesulfonic acid).

After cooling to 25°C, said first mixture was subjected to extraction with 1 1 of a mixture of toluene and n-butanol (3/1 by volume).

The mixture of toluene and n-butanol was subsequently separated and evaporated, at reduced pressure, obtaining a further solid phase comprising 19.5 g (dry weight) of 2-naphthalenesulfonic acid (recovery yield 98% calculated with respect to the total amount of the acid present in the aqueous solution).

After separation of the acid, glacial acetic acid was added to said first mixture until a pH of said first aqueous phase equal to 5.0 was obtained.

An aqueous solution of the enzyme Celluclast 1,5L (Novozymes) corresponding to 1400 FPU (Filter Paper Units) and the enzyme Novozym 188 (from Novozymes) corresponding to 18000 BGU (Beta Glucanase Units) was subsequently added. The suspension thus obtained was maintained under stirring, for 72 hours, at 45°C.

At the end of the enzymatic hydrolysis, 80 g (dry weight) of a second solid phase comprising lignin (65 g - dry weight), non-hydrolyzed cellulose (13 g - dry weight) and cellular debris (2 g - dry weight) and 1,220 g of a second aqueous phase comprising glucose (139 g - dry weight), mannose (9 g - dry weight), xylose (67 g - dry weight) and amino acids (20 g - dry weight), were separated by filtration.

Said second aqueous phase can be subjected to fermentation operating as described in Example 1.

## Claims

1. A process for the production of lipids from biomass including at least one polysaccharide comprising:
- subjecting said biomass to acid hydrolysis in the presence of an aqueous solution of at least one organic acid selected from alkyl- or aryl-sulfonic acids having from C₇ to C₂₀ carbon atoms, at a temperature ranging from 80°C to 160°C, obtaining a first mixture comprising a first solid phase and a first aqueous phase;
- subjecting said first mixture to enzymatic hydrolysis obtaining a second mixture comprising a second solid phase and a second aqueous phase;
- subjecting said second aqueous phase to fermentation in the presence of at least one oleaginous yeast obtaining an oleaginous cellular biomass comprising lipids.

2. The process according to claim 1, wherein said alkyl-or aryl-sulfonic acids have from C₉ to C₁₅ carbon atoms.

3. The process according to claim 1 or 2, wherein said temperature ranges from 100°C to 150°C.

4. The process according to any one of the preceding claims, wherein said polysaccharide is selected from cellulose, hemicellulose, or their mixtures.

5. The process according to any one of the preceding claims, wherein said biomass is a lignocellulosic biomass.

6. The process according to claim 5, wherein said lignocellulosic biomass is selected from:
- products of cultures expressly cultivated for energy use including by-products, residues and wastes of said cultures or of their processing;
- products of agricultural cultivations, forestation and silviculture, comprising wood, plants, residues and by-products of agricultural processing, forestation and silviculture;
- agri-foodstuffs by-products intended for human feeding or zootechnics;
- residues, not chemically treated, of the paper industry;
- waste products coming from the differentiated collection of solid urban waste.

7. The process according to any one of the preceding claims, wherein said biomass is subjected to a preliminary grinding process before being subjected to acid hydrolysis.

8. The process according to claim 7, wherein said biomass is ground until particles having a diameter ranging from 0.1 mm to 10 mm are obtained.

9. The process according to claim 8, wherein said biomass is ground until particles having a diameter lower than 1 mm are obtained.

10. The process according to any one of the preceding claims, wherein said biomass is present in the reaction mixture in an amount ranging from 5% by weight to 40% by weight with respect to the total weight of the reaction mixture.

11. The process according to any one of the preceding claims, wherein said at least one organic acid is soluble in water and extractable by means of an organic solvent insoluble in water.

12. The process according to any one of the preceding claims, wherein said alkyl- or aryl sulfonic acids are selected from: dodecyl-sulfonic acid, para-toluene-sulfonic acid, 1-naphthalene-sulfonic acid, 2-naphthalene-sulfonic acid, 1,5-naphthalene-disulfonic acid, or their mixtures.

13. The process according to claim 12, wherein said at least one organic acid is selected from para-toluene-sulfonic acid, 2-naphthalene-sulfonic acid, 1,5-naphthalene-disulfonic acid, or their mixtures.

14. The process according to any one of the preceding claims, wherein said at least one organic acid is present in the aqueous solution at a concentration ranging from 0.1% by weight to 5% by weight with respect to the total weight of the aqueous solution.

15. The process according to any one of the preceding claims, wherein said acid hydrolysis is carried out for a time ranging from 20 minutes to 6 hours.

16. The process according to any one of the preceding claims, wherein said first solid phase comprises lignin and cellulose.

17. The process according to any one of the preceding claims, wherein said first aqueous phase comprises at least one sugar having from C₅ to C₆ carbon atoms and said at least one organic acid.

18. The process according to claim 17, wherein said at least one sugar is xylose.

19. The process according to any one of the preceding claims, wherein said first mixture is subjected to extraction with an organic solvent insoluble in water.

20. The process according to claim 19, wherein said organic solvent insoluble in water is selected from: halogenated hydrocarbons; aromatic hydrocarbons; aliphatic alcohols having from C₄ to C₆ carbon atoms; or mixtures thereof.

21. The process according to claim 20, wherein said organic solvent insoluble in water is selected from mixtures of toluene and n-butanol.

22. The process according to any one of the claims from 19 to 21, wherein said organic solvent insoluble in water is evaporated obtaining a further solid phase comprising said at least one organic acid.

23. The process according to claim 22, wherein said process comprises reutilizing said at least one organic acid in said acid hydrolysis.

24. The process according to any one of the preceding claims, wherein said second solid phase comprises lignin.

25. The process according to any one of the preceding claims, wherein said second aqueous phase comprises at least one sugar having from C₅ to C₆ carbon atoms.

26. The process according to any one of the preceding claims, wherein said second aqueous phase comprises xylose, glucose and cellobiose.

27. The process according to any one of the preceding claims, wherein said fermentation is carried out at a temperature ranging from 20°C to 40°C.

28. The process according to any one of the preceding claims, wherein said fermentation is carried out for a time ranging from 3 days to 10 days.

29. The process according to any one of the preceding claims, wherein said fermentation is carried out at a pH ranging from 4.5 to 6.5.

30. The process according to any one of the preceding claims, wherein said oleaginous yeast is selected from: *Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Pichia stipitis, Torulopsis sp..*

31. The process according to any one of the preceding claims, wherein said fermentation is a feed-batch fermentation.

32. The process according to any one of the preceding claims, wherein said oleaginous cellular biomass is subjected to mechanical treatment.

33. The process according to claim 32, wherein said mechanical treatment is carried out using a "French press cell", operating at a pressure ranging from 20 Kpsi to 50 Kpsi.

34. The process according to claim 32 or 33, wherein said oleaginous cellular biomass is subjected to centrifugation after mechanical treatment.

35. The process according to claim 34, wherein said centrifugation is carried out for a period of time ranging from 5 minutes to 30 minutes.

36. The process according to claim 34 or 35, wherein said centrifugation is carried out at a rotational speed ranging from 3,000 rpm to 9,000 rpm.

37. The process according to any one of the claims from 34 to 36, wherein at the end of the centrifugation the following three phases are obtained:
(i) an oily phase comprising lipids;
(ii) an aqueous phase comprising traces of non-separated lipids and non-fermented sugars;
(iii) a wet solid phase comprising cellular debris and traces of non-separated lipids.

38. The process according to any one of the preceding claims, wherein said wet solid phase (iii) is conveyed to the acid hydrolysis.

39. The process according to any one of the preceding claims, wherein said aqueous phase (ii) and/or said wet solid phase (iii) are subjected to extraction with at least one solvent which is selected from aliphatic hydrocarbons having from C₃ to C₁₀ carbon atoms; or with a mixture comprising at least one aliphatic alcohol having from C₃ to C₅ carbon atoms and at least one aliphatic hydrocarbon having from C₃ to C₁₀ carbon atoms.

## Patentansprüche

1. Verfahren zur Herstellung von Lipiden aus Biomasse einschließend mindestens ein Polysacharid umfassend:
- Unterwerfung der Biomasse mit einer Säurehydrolyse in Gegenwart einer wässrigen Lösung mindestens einer organischen Säure ausgewählt aus Alkyl-oder Aryl-Sulfonsäuren mit C₇ bis C₂₀ Kohlenstoffatomen bei einer Temperatur in einem Bereich von 80 °C bis 160 °C, erhaltend eine erste Mischung umfassend eine erste feste Phase und eine erste wässrige Phase;
- Unterwerfung der ersten Mischung mit enzymatischer Hydrolyse, erhaltend eine zweite Mischung umfassend eine zweite feste Phase und eine zweite wässrige Phase;
- Unterwerfung der zweiten wässrigen Phase mit Fermentierung in Gegenwart mindestens einer öligen Hefe, erhaltend eine ölige Zell-Biomasse umfassend Lipide.

2. Verfahren gemäß Anspruch 1, worin die Alkyl- oder Aryl-Sulfonsäuren C₉ bis C₁₅ Kohlenstoffatome haben.

3. Verfahren gemäß Anspruch 1 oder 2, worin die Temperatur in einem Bereich von 100 °C bis 150 °C liegt.

4. Verfahren gemäß einem der vorstehenden Ansprüche, worin das Polysacharid ausgewählt ist aus Zellulose, Hemizellulose oder deren Mischungen.

5. Verfahren gemäß irgendeinem der vorstehenden Ansprüche, worin die Biomasse eine Lignozellulose-Biomasse ist.

6. Verfahren gemäß Anspruch 5, worin die Lignozellulose-Biomasse ausgewählt ist aus:
- Produkte aus Kulturen die ausdrücklich zur Energieverwendung kultiviert wurden einschließlich der Nebenprodukte, Reste und Rückstände der Kulturen oder aus deren Verarbeitung;
- Produkte aus Agrikulturkultivierungen, Aufforstung und Waldwirtschaft, umfassend Holz, Pflanzen, Reste und Nebenprodukte aus Agrikulturverarbeitung, Aufforstung und Waldwirtschaft;
- Agri-Nahrungsmittelnebenprodukte beabsichtigt für menschliche Ernährung oder Zootechnik;
- Reste, nicht-chemisch behandelt, aus der Papierindustrie;
- Abfallprodukte, die aus der differenzierten Sammlung von festen städtischen Abfällen herrühren.

7. Verfahren gemäß irgendeinem der vorstehenden Ansprüche, worin die Biomasse einem vorläufigen Mahlverfahren unterworfen wurde bevor sie der Säurehydrolyse unterzogen wurde.

8. Verfahren gemäß Anspruch 7, worin die Biomasse gemahlen wird bis Teilchen mit einem Durchmesser in einem Bereich von 0,1 mm bis 10 mm erhalten wurden.

9. Verfahren gemäß Anspruch 8, worin die Biomasse gemahlen wird bis Teilchen mit einem Durchmesser geringer als 1 mm erhalten wurden.

10. Verfahren gemäß irgendeinem der vorstehenden Ansprüche, worin die Biomasse in der Reaktionsmischung in einer Menge in einem Bereich von 5 Gew. % bis 40 Gew. % hinsichtlich des Gesamtgewichts der Reaktionsmischung vorliegt.

11. Verfahren gemäß irgendeinem der vorstehenden Ansprüche, worin die mindestens eine organische Säure in Wasser löslich ist und mittels eines organischen Lösemittels, das in Wasser unlöslich ist, extrahierbar ist.

12. Verfahren gemäß irgendeinem der vorstehenden Ansprüche, worin die Alkyl-oder Aryl-Sulfonsäuren ausgewählt sind aus: Dodecyl-Sulfonsäure, para-Toluen-Sulfonsäure, 1-Naphthalin-Sulfonsäure, 2-Naphthalin-Sulfonsäure, 1,5-Naphthalin-Disulfonsäure oder deren Mischungen.

13. Verfahren gemäß Anspruch 12, worin die mindestens eine organische Säure ausgewählt ist aus Para-Toluen-Sulfonsäure, 2-Naphthalin-Sulfonsäure, 1,5-Naphthalin-Disulfonsäure oder deren Mischungen.

14. Verfahren gemäß irgendeinem der vorstehenden Ansprüche, worin die mindestens eine organische Säure in der wässrigen Lösung bei einer Konzentration in einem Bereich von 0,1 Gew. % bis 5 Gew. %, hinsichtlich des Gesamtgewichts der wässrigen Lösung, vorliegt.

15. Verfahren gemäß irgendeinem der vorstehenden Ansprüche, worin die Säure-Hydrolyse über einen Zeitraum durchgeführt wird in einem Bereich von 20 Minuten bis 6 Stunden.

16. Verfahren gemäß irgendeinem der vorstehenden Ansprüche, worin die erste feste Phase Lignin und Zellulose umfasst.

17. Verfahren gemäß irgendeinem der vorstehenden Ansprüche, worin die erste wässrige Phase mindestens einen Zucker mit C₅ bis C₆ Kohlenstoffatomen und die mindestens eine organische Säure umfasst.

18. Verfahren gemäß Anspruch 17, worin der mindestens eine Zucker Xylose ist.

19. Verfahren gemäß irgendeinem der vorstehenden Ansprüche, worin die erste Mischung einer Extraktion mit einem organischen Lösemittel, unlöslich in Wasser, unterworfen wird.

20. Verfahren gemäß Anspruch 19, worin das organische Lösemittel, unlöslich in Wasser, ausgewählt ist aus: halogenierten Kohlenwasserstoffen, aromatischen Kohlenwasserstoffen, aliphatischen Alkoholen mit C₄ bis C₆ Kohlenstoffatomen oder Mischungen davon.

21. Verfahren gemäß Anspruch 20, worin das organische Lösemittel, unlöslich in Wasser, ausgewählt ist aus Mischungen von Toluen und n-Butanol.

22. Verfahren gemäß irgendeinem der Ansprüche 19 bis 21, worin das organische Lösemittel, unlöslich in Wasser, verdampft wird, erhaltend eine weitere feste Phase umfassend mindestens eine organische Säure.

23. Verfahren gemäß Anspruch 22, worin das Verfahren die Wiederverwendung der mindestens einen organischen Säure in der Säure-Hydrolyse umfasst.

24. Verfahren gemäß irgendeinem der vorstehenden Ansprüche, worin die zweite feste Phase Lignin umfasst.

25. Verfahren gemäß irgendeinem der vorstehenden Ansprüche, worin die zweite wässrige Phase mindestens einen Zucker mit C₅ bis C₆ Kohlenstoffatomen umfasst.

26. Verfahren gemäß irgendeinem der vorstehenden Ansprüche, worin die zweite wässrige Phase Xylose, Glukose und Cellobiose umfasst.

27. Verfahren gemäß irgendeinem der vorstehenden Ansprüche, worin die Fermentierung bei einer Temperatur in einem Bereich von 20 °C bis 40 °C durchgeführt wird.

28. Verfahren gemäß irgendeinem der vorstehenden Ansprüche, worin die Fermentation über einen Zeitraum in einem Bereich von 3 Tagen bis 10 Tagen ausgeführt wird.

29. Verfahren gemäß irgendeinem der vorstehenden Ansprüche, worin die Fermentation bei einem pH in einem Bereich von 4,5 bis 6,5 durchgeführt wird.

30. Verfahren gemäß irgendeinem der vorstehenden Ansprüche, worin die ölige Hefe ausgewählt ist aus: *Rhodotorula glutinis, Rhodotorula gracilis, Rhodotorula graminis, Lypomices starkeyi, Lypomices lipofer, Trigonopsis variabilis, Candida kefyr, Candida curvata, Candida lipolytica, Pichia stipitis, Torulopsis sp.*

31. Verfahren gemäß irgendeinem der vorstehenden Ansprüche, worin die Fermentierung eine Feed-Batch-Fermentierung ist.

32. Verfahren gemäß irgendeinem der vorstehenden Ansprüche, worin die ölige Zell-Biomasse einer mechanischen Behandlung unterworfen wird.

33. Verfahren gemäß Anspruch 32, worin die mechanische Behandlung ausgeführt wird mit einer "French press cell", die bei einem Druck im Bereich von 20 Kpsi bis 50 Kpsi operiert.

34. Verfahren gemäß Anspruch 32 oder 33, worin die ölige Zell-Biomasse einer Zentrifugierung nach der mechanischen Behandlung unterworfen wird.

35. Verfahren gemäß Anspruch 34, worin die Zentrifugierung über einen Zeitraum in einem Bereich von 5 Minuten bis 30 Minuten durchgeführt wird.

36. Verfahren gemäß Anspruch 34 oder 35, worin die Zentrifugierung bei einer Umdrehungsgeschwindigkeit in einem Bereich von 3000 rpm bis 9000 rpm durchgeführt wird.

37. Verfahren gemäß irgendeinem der Ansprüche 34 bis 36, worin am Ende der Zentrifugierung die folgenden drei Phasen erhalten werden:
(i) eine ölige Phase umfassend Lipide;
(ii) eine wässrige Phase umfassend Spuren von nichtgetrennten Lipiden und nicht-fermentierten Zuckern;
(iii) eine feuchte feste Phase umfassend zelluläre Rückstände und Spuren nicht-getrennter Lipide.

38. Verfahren gemäß irgendeinem der vorstehenden Ansprüche, worin die feuchte feste Phase (iii) der Säure-Hydrolyse zugeführt wird.

39. Verfahren gemäß irgendeinem der vorstehenden Ansprüche, worin die wässrige Phase (ii) und/oder die feuchte feste Phase (iii) einer Extraktion mit mindestens einem Lösemittel unterworfen wird, welches ausgewählt ist aus aliphatischen Kohlenwasserstoffen mit C₃ bis C₁₀ Kohlenstoffatomen; oder mit einer Mischung umfassend mindestens einen aliphatischen Alkohol mit C₃ bis C₅ Kohlenstoffatomen und mindestens einem aliphatischen Kohlenwasserstoff mit C₃ bis C₁₀ Kohlenstoffatomen.

## Revendications

1. Procédé de production de lipides à partir de biomasse comprenant au moins un polysaccharide, lequel procédé comporte les étapes suivantes :
- soumettre ladite biomasse à une hydrolyse acide, en présence d'une solution aqueuse d'au moins un acide organique choisi parmi les acides alcane-sulfoniques ou arène-sulfoniques comportant de C₇ à C₂₀ atomes de carbone, à une température de 80 °C à 160 °C, et obtenir ainsi un premier mélange comprenant une première phase solide et une première phase aqueuse ;
- soumettre ledit premier mélange à une hydrolyse enzymatique, et obtenir ainsi un deuxième mélange comprenant une deuxième phase solide et une deuxième phase aqueuse ;
- et soumettre ladite deuxième phase aqueuse à une fermentation, en présence d'au moins une levure oléagineuse, et obtenir ainsi une biomasse cellulaire oléagineuse comprenant des lipides.

2. Procédé conforme à la revendication 1, dans lequel lesdits acides alcane-sulfoniques ou arène-sulfoniques comportent de C₉ à C₁₅ atomes de carbone.

3. Procédé conforme à la revendication 1 ou 2, dans lequel ladite température vaut de 100 °C à 150 °C.

4. Procédé conforme à l'une des revendications précédentes, dans lequel ledit polysaccharide est choisi parmi de la cellulose, de l'hémicellulose et leurs mélanges.

5. Procédé conforme à l'une des revendications précédentes, dans lequel ladite biomasse est une biomasse lignocellulosique.

6. Procédé conforme à la revendication 5, dans lequel ladite biomasse lignocellulosique est choisie parmi :
- des produits de cultures cultivées exprès pour usage énergétique, y compris les sous-produits, résidus et déchets de ces cultures ou de leur traitement ;
- des produits de cultures agricoles, d'exploitation de forêts ou de sylviculture, y compris du bois, des végétaux entiers, des résidus et des sous-produits d'exploitation agricole, d'exploitation de forêts ou de sylviculture ;
- des sous-produits agro-alimentaires destinés à l'alimentation humaine ou à la zootechnie ;
- des résidus de l'industrie papetière qui n'ont pas subi de traitement chimique ;
- des déchets provenant de collecte et tri de déchets urbains solides.

7. Procédé conforme à l'une des revendications précédentes, dans lequel ladite biomasse est soumise à une opération préliminaire de broyage avant de subir une hydrolyse acide.

8. Procédé conforme à la revendication 7, dans lequel ladite biomasse est broyée jusqu'à l'obtention de particules dont le diamètre vaut de 0,1 mm à 10 mm.

9. Procédé conforme à la revendication 8, dans lequel ladite biomasse est broyée jusqu'à l'obtention de particules dont le diamètre vaut moins de 1 mm.

10. Procédé conforme à l'une des revendications précédentes, dans lequel ladite biomasse est présente dans le mélange réactionnel en une proportion de 5 % à 40 %, en poids rapporté au poids total du mélange réactionnel.

11. Procédé conforme à l'une des revendications précédentes, dans lequel ledit acide organique au nombre d'au moins un est soluble dans l'eau et peut en être extrait au moyen d'un solvant organique insoluble dans l'eau.

12. Procédé conforme à l'une des revendications précédentes, dans lequel lesdits acides alcane-sulfoniques ou arène-sulfoniques sont choisis parmi les suivants : acide dodécane-sulfonique, acide para-toluène-sulfonique, acide naphtalène-1-sulfonique, acide naphtalène-2-sulfonique et acide naphtalène-1,5-disulfonique, et leurs mélanges.

13. Procédé conforme à la revendication 12, dans lequel ledit acide organique au nombre d'au moins un est choisi parmi l'acide para-toluène-sulfonique, l'acide naphtalène-2-sulfonique et l'acide naphtalène-1,5-disulfonique, et leurs mélanges.

14. Procédé conforme à l'une des revendications précédentes, dans lequel ledit acide organique au nombre d'au moins un est présent dans la solution aqueuse en une concentration de 0,1 à 5 %, en poids rapporté au poids total de la solution aqueuse.

15. Procédé conforme à l'une des revendications précédentes, dans lequel ladite hydrolyse acide est poursuivie durant un laps de temps de 20 minutes à 6 heures.

16. Procédé conforme à l'une des revendications précédentes, dans lequel ladite première phase solide comprend de la lignine et de la cellulose.

17. Procédé conforme à l'une des revendications précédentes, dans lequel ladite première phase aqueuse comprend au moins un sucre comportant de C₅ à C₉ atomes de carbone, ainsi que ledit acide organique au nombre d'au moins un.

18. Procédé conforme à la revendication 17, dans lequel ledit sucre au nombre d'au moins un est du xylose.

19. Procédé conforme à l'une des revendications précédentes, dans lequel ledit premier mélange est soumis à une extraction opérée avec un solvant organique insoluble dans l'eau.

20. Procédé conforme à la revendication 19, dans lequel ledit solvant organique insoluble dans l'eau est choisi parmi les hydrocarbures halogénés, les hydrocarbures aromatiques, les alcools aliphatiques comportant de C₄ à C₆ atomes de carbone, et les mélanges de tels solvants.

21. Procédé conforme à la revendication 20, dans lequel ledit solvant organique insoluble dans l'eau est choisi parmi des mélanges de toluène et de n-butanol.

22. Procédé conforme à l'une des revendications 19 à 21, dans lequel on évapore ledit solvant organique insoluble dans l'eau, et l'on obtient ainsi une phase solide supplémentaire comprenant ledit acide organique au nombre d'au moins un.

23. Procédé conforme à la revendication 22, lequel procédé comporte le fait de réutiliser ledit acide organique au nombre d'au moins un dans ladite hydrolyse acide.

24. Procédé conforme à l'une des revendications précédentes, dans lequel ladite deuxième phase solide comprend de la lignine.

25. Procédé conforme à l'une des revendications précédentes, dans lequel ladite deuxième phase aqueuse comprend au moins un sucre comportant de C₅ à C₆ atomes de carbone.

26. Procédé conforme à l'une des revendications précédentes, dans lequel ladite deuxième phase aqueuse comprend du xylose, du glucose et du cellobiose.

27. Procédé conforme à l'une des revendications précédentes, dans lequel ladite fermentation s'effectue à une température valant de 20 °C à 40 °C.

28. Procédé conforme à l'une des revendications précédentes, dans lequel ladite fermentation se poursuit durant un laps de temps de 3 jours à 10 jours.

29. Procédé conforme à l'une des revendications précédentes, dans lequel ladite fermentation s'effectue à un pH de 4,5 à 6,5.

30. Procédé conforme à l'une des revendications précédentes, dans lequel ladite levure oléagineuse est choisie parmi les suivantes : *Rhodotorula glutinis, Rhodotorula gracilis, Rhodotorula graminis, Lipomyces starkeyi, Lipomyces lipofer, Trigonopsis variabilis, Candida kefyr, Candida curvata, Candida lipolytica, Pichia stipitis,* et *Torulopsis sp..*

31. Procédé conforme à l'une des revendications précédentes, dans lequel ladite fermentation est une fermentation en mode discontinu.

32. Procédé conforme à l'une des revendications précédentes, dans lequel on soumet ladite biomasse cellulaire oléagineuse à un traitement mécanique.

33. Procédé conforme à la revendication 32, dans lequel ledit traitement mécanique est réalisé au moyen d'une presse à cellules dite « presse de French » fonctionnant à une pression valant de 20 Kpsi à 50 Kpsi.

34. Procédé conforme à la revendication 32 ou 33, dans lequel, après le traitement mécanique, on soumet ladite biomasse cellulaire oléagineuse à une centrifugation.

35. Procédé conforme à la revendication 34, dans lequel ladite centrifugation est poursuivie durant un laps de temps de 5 minutes à 30 minutes.

36. Procédé conforme à la revendication 34 ou 35, dans lequel ladite centrifugation est effectuée à une vitesse de rotation valant de 3 000 t/min à 9 000 t/min.

37. Procédé conforme à l'une des revendications 34 à 36, dans lequel on obtient, à la fin de la centrifugation, les trois phases suivantes :
i) une phase huileuse comprenant des lipides,
ii) une phase aqueuse comprenant des traces de lipides non-séparés et de sucres non-fermentés,
iii) et une phase solide humide comprenant des débris de cellules et des traces de lipides non-séparés.

38. Procédé conforme à l'une des revendications précédentes, dans lequel ladite phase solide humide (iii) est renvoyée à l'hydrolyse acide.

39. Procédé conforme à l'une des revendications précédentes, dans lequel on soumet ladite phase aqueuse (ii) et/ou ladite phase solide humide (iii) à une extraction opérée avec au moins un solvant qui est choisi parmi les hydrocarbures aliphatiques comportant de C₃ à C₁₀ atomes de carbone, ou avec un mélange comprenant au moins un alcool aliphatique comportant de C₃ à C₅ atomes de carbone et au moins un hydrocarbure aliphatique comportant de C₃ à C₁₀ atomes de carbone.
